# EUROPEAN PATENT SPECIFICATION

(11) **EP 2 473 209 B1**
(45) Date of publication and mention of the grant of the patent: **14.10.2015**
(21) Application number: 10763584.9
(22) Date of filing: 22.07.2010
(51) Int. Cl.: A23L 1/304, A23L 1/305, A61L 27/36

(54) **THE PRODUCT INTENDED FOR FACILITATION OF BONE OSSIFICATION, THE METHOD OF ITS MANUFACTURE AND USE**
PRODUKT ZUR FÖRDERUNG VON VERKNÖCHERUNG, VERFAHREN ZU SEINER HERSTELLUNG UND VERWENDUNG
PRODUIT DESTINÉ À FACILITER L OSSIFICATION DES OS, PROCÉDÉ DE FABRICATION ET UTILISATION

(30) Priority: 12.08.2009 CZ 20090540
(43) Date of publication of application: 11.07.2012
(73) Proprietor: Hypro Otrokovice S.R.O., 75602 Otrokovice (CZ)
(72) Inventor: GALATÍK, Antonín, 76502 Otrokovice (CZ)
(74) Representative: Kreizlova, Dana
(86) International application number: PCT/CZ2010/000081
(87) International publication number: WO 2011/018057

(56) References cited:
- WO-A1-00/47132
- WO-A1-2010/054527
- US-A- 4 314 380
- US-A- 5 246 457

## Description

### Scope of Technology

The invention is related to the product intended for facilitation of bone ossification, which is usable for implantation in bone defects, in controlled bone regeneration, filling of bone cysts and smaller missing parts of the bones or as a food supplement that supports ossification.

### State of the Art

A common clinical procedure of implantation of the osteo-inductive, i.e. bone formation supporting materials, uses so-called autograft where the needed part of bone is removed from another place of the patient's body. However, the amount of bone which can removed from the patient is limited and the own approach is considerably invasive. Less traumatic option is to use allograft when the bone is taken from a deceased donor. This process is somewhat problematic due to the risk of transmission of infectious diseases whereas these implants contain only a small amount of growth factors because they do not contain living cells. To avoid the risk of infection an array of various artificial products have been developed and marketed which substitute human bone implants. Several procedures for preparing of collagen-hydroxyapatite composites for surgical treatment of bone defects have been described. So as to obtain hydroxyapatite some procedures use bones of vertebrates and animal collagen, mainly of type I, which is most often obtained from animal fibrous tissues.

**Among them mainly a procedure according to document** WO 0047132 **shall be mentioned which is based on the process of preparing a xenograft formed of a soft or bone tissue for implantation into a human. The method uses the soft or bone tissue from a non-human animal to provide a xenograft via treatment of this animal material so as to adapt them to the needs of human body.**

**Another method based on similar principle is a technology described in document** US 5,246,457**. Xenogeneic collagen/mineral preparations attended for bone repair are created from calcium phosphate (94%) mineral component containing hydroxyapatite and tri-calcium phosphate (60.40) and atelopeptide reconstituted fibrillar collagen. The said material is subjected to further processing finished by molding and lyophilizing the composition in the mold.**

The hydroxyapatite osteo-inductive compounds are commercially available also as the products isolated from the heat-modified mineral material of sea corals, or they are produced synthetically by precipitation of calcium and phosphate ions in aqueous ammonia environment.

The problem of these synthetic hydroxyapatite products can be their cytotoxicity caused by ammonia and ammonium compounds from the reaction environment, which can be very difficult to remove from the product. The ratio of calcium and phosphorus (Ca/P) in the synthetic products may differ from the native bones, and is usually in the range from 1.62 to 1.69.

The mineral apatite component is, with the advantage, created by precipitation of calcium compounds by phosphates in aqueous collagen dispersions. In such created composites the orientation of collagen macromolecules and the longitudinal axis of the hydroxyapatite microcrystals can be to some extent identical which can be controlled by adjusting the acidity and temperature of the precipitation environment. However, the exact replica of the native composite, which is a structurally inclusion compound of clactrate type is unreachable by the synthetic route. Many manufacturers produce mineral sintered compounds, i.e. heat treated; however they differ chemically from the biological carbonate form of apatite which can found in bones. It was found that these adjusted phosphate salts were less degradable compared to the natural carbonate forms. A particular construction of such bone structure can be found in the document US 4,314,380 - it contains a core of porous, organic-free hydroxyapatite; such core being impregnated and coated with atelo-collagen. The hydroxyapatite material of the core has been obtained from chemically-treated, burned, baked animal bone. Despite the sophisticated construction such artificial bone is loaded by already mentioned disadvantages of all known artificial bones - they hardly become equal to native bone and, moreover, due to heat treating they increasingly differ from the native material. On the other hand, the complicated structure described here demands a costly technology of its production which is further appreciable disadvantage of the artificial bone described in US 4,314,380**.**

Another deficiency of the known solutions which are based on the natural composites - such as above mentioned procedure according to document WO 0047132 **-** is the antigenicity of animal collagen though it is only mild in majority of patients. The reasons of the antigenicity are the antigenic determinants, i.e. specific amino-acid sequences in collagen which are different in various animal species. They are located mainly in the telopeptidic terminal areas of the collagen protein. However, this disadvantage can become a serious problem in patients with allergy to foreign proteins. A disposition of the patient to increased or even allergic reaction to a foreign collagen may not be known before the surgery and this risk is, as well as the proportion of people with allergies in the population due to lifestyle factors increasing. The same problems and deficiencies can be related to the solution according to document WO 2010054527 which describes a method of preparing a jawbone implant from bovine or porcine jawbones, shaping and treatment of such material so as to remove antigens, adapting it to the needs of a human body and applying the product in human jawbone surgery. The possible problems with some allergic reactions mentioned above are evident.

For type I collagen which is used for the preparation of absorbable haemostatics the allergen telopeptides can be eliminated using the specific enzymatic process called atelopeptidation. However, the atelopeptidic modification of collagen in the native bone composites has not been described yet. Only preparation of a synthetic bone composite using a modified atelocollagen instead of collagen is known.

### Ground of the Invention

The product for facilitation of bone ossification according to the invention contributes to elimination of the above stated deficiencies of the known technology status which consists of the native composite of the hydroxyapatite structure with collagen. A principle of the invention lies in the fact that the collagen contained in the native bone composite is modified, at least partially devoid of the terminal antigenic telopeptides, which is a reaction product of enzymatic atelopeptidation.

Besides the basic ingredient - the native bone composite with modified collagen, the product may, as the advantage, further contain at least one excipient from the group comprising growth factors, antibiotics, bacteriostatic or pharmacologically active substances.

The principle of the method of manufacture of the product that facilitates bone ossification according to the invention lies in the fact that proteolytic enzymes are applied on the purified bone mash and these enzymes catalyze hydrolysis of terminal collagen telopeptides and so they modify collagen by means of atelopeptidation.

It is at the same time advantageous if the bone structures and non-collagen organic components are removed from the bone mash before atelopeptidation.

A proteolytic enzyme without any collagenolytic activity, preferably pepsin, should be used with the advantage for atelopeptidation.

The bone composite of the invention combines the properties of the preserved natural bone mineral structure and collagen component with the advantage of the suppressed antigenicity of the used animal collagen. This new effect is, as already mentioned, achieved by atelopeptidation, i.e. by hydrolysis of the collagen telopeptides catalyzed by the appropriate specific proteolytic enzymes, which are chosen so as not to damage the main triple helical areas of collagen. The atelopeptidic reaction is, with the advantage, carried out on the purified bone mash from which other non- collagen organic components had been previously removed. The modified collagen is present in the bone mash in the insoluble form and the process of atelopeptidation has the character of heterogeneous reaction. The area of a phase boundary is important for the kinetics of the heterogeneous reactions. The bone structure is porous, has a relatively large internal surface and its capillary network is permeable for enzymes; despite it, its disintegration into smaller particles is however convenient which increases the interphase surface, decreases the diffusion distances for reactants and accelerate the progress of the enzymatic reactions. Using of the product that facilitates ossification of bones of the invention may lie in its surgical implantation in the processes of controlled bone regeneration or for filling of bone cysts and smaller missing parts of bones or in the application of this product as a food supplement that supports ossification.

Another potential use of the product of the invention is the administration as a food supplement to support bone ossification in persons at the time of increased demands for growth or in people with osteoporosis, arthritis and other degenerative illnesses.

### Examples

### Example 1

An example of the product of the invention is a sterile composite of the native hydroxyapatite and atelocollagen - i.e. the collagen from which at least the terminal antigen telopeptides had been removed which is a reaction product of the enzymatic atelopeptidation - in the natural structural complex.

The manufacturing process of the atelocollagen bone composite in the exemplary performance consists in the separation of the central part of the tarsal bovine bones, cleaning of their surface and marrow cavity, crushing into the small grit with an average particle size of 4.0 to 0.1 mm, extraction of fat components using gasoline, drying under reduced pressure, repeated extraction of cellular globular proteins by 10% aqueous sodium chloride solution, followed by extraction with aqueous solutions of diluted acetic acid and then by atelopeptidation which consists in incubation of the grit suspension acidified to pH 2.5 -3.0 with pepsin of pharmaceutical purity pepsin at 38<0>C. It is then followed by flow washing using deionized water to the constant water conductivity value, centrifuging of the suspension followed by freezing the grit and its frost drying under at low pressure. The resulting pulp is filled in the packaging, hermetically sealed and sterilized by gamma radiation at a dose of 25kGy.

### Example 2

The composite prepared by the procedure identical to example 1 is after its sterilization under aseptic conditions supplemented by the growth factor IGF-I in a quantity of 2.5 micrograms per one gram of the composite and by the secondary sodium phosphate in a quantity of 25 milligrams per gram of the composite to control pH value. The resulting mixture is homogenized by stirring, filled in and hermetically sealed in containers.

### Example 3

An example of the composite intended for use as a food supplement is the product prepared according to example 1, which is supplemented with vitamin C in quantity of 50 mg per one gram and by the complex of vitamin B₁ in quantity of 7 mg per one gram of the product before filling, homogenized by stirring the mixture, filled in the packaging and hermetically sealed.

## Claims

1. The product for facilitation bone ossification of bone defect on the basis of native bone composite which contains **the hydroxyapatite structure** and modified collagen component, **characterized by the fact** that the **hydroxyapatite structure** comprising purified bone mash and collagen contained in the native bone composite is modified, at least partially devoid of the terminal antigenic telopeptides.

2. The product according to the claim 1 is **characterized by** the fact that besides the basic ingredient - the.native bone composite with modified collagen contains at least one excipient from the group comprising growth factors, antibiotics, bacteriostatic or pharmacologically active substances.

3. The method of manufacture of the product that facilitates bone ossification according to claim 1 is **characterized by** the fact that the enzymes are applied on the purified bone mash and these enzymes catalyze hydrolysis of terminal collagen telopeptides and so they modify collagen by means of atelopeptidation.

4. The method according to the claim 3 is **characterized by** the fact that the bone structures and non-collagen organic components are removed from the bone mash before atelopeptidation.

5. The method according to claim 3 or 4 is.**characterized by** the fact that a proteolytic enzyme without any collagenolytic activity is used for atelopeptidation.

6. The method according to claim 5 is **characterized by** the fact that the used proteolytic enzyme is pepsin.

7. The method according to claims 3 to 6 is **characterized by** the fact that the product after atelopeptidation is subsequently washed by deionized water until constant conductivity values, centrifuged, frozen and dried by sublimation at low pressures, possibly supplemented by the added excipient, or excipients, formed by pressing in the forms, or filled in and sterilized by gamma radiation.

8. The product according to claim 1 for use in a treatment of bone defects and wherein the product is implanted as bone filler for bone cysts and smaller missing parts of bones.

9. The product according to claim 1 for use in the treatment of osteoporosis, arthritis and other degenerative illnesses, and wherein the product is administered as food supplement.

## Patentansprüche

1. Ein Produkt zur Vereinfachung der Ossifikation bei Knochendefekten, das auf nativem Knochenverbundmaterial beruht, welches eine Hydroxylapatit-Struktur sowie modifizierte Kollagenbausteine enthält, **dadurch gekennzeichnet, dass** die Hydroxylapatit-Struktur, welche gereinigten Knochenbrei enthält, und das Kollagen, das in dem nativen Knochenverbundmaterial enthalten ist, modifiziert wurden und zumindest teilweise frei von den terminalen antigenischen Telopeptiden sind.

2. Das Produkt nach Anspruch 1, **dadurch gekennzeichnet, dass** das native Knochenverbundmaterial mit modifiziertem Kollagen neben dem grundlegenden Element mindestens einen Hilfsstoff aus der Gruppe enthält, die Wachstumsfaktoren, Antibiotika, bakteriostatische oder pharmakologische Wirkstoffe umfassen.

3. Eine Methode für Herstellung des Produkts zur Vereinfachung der Ossifikation nach Anspruch 1, **dadurch gekennzeichnet, dass** die Enzyme auf den gereinigten Knochenbrei aufgetragen werden und diese Enzyme die Hydrolyse von terminalen Kollagen-Telopeptiden katalysieren und auf diese Weise Kollagen durch Atelopeptidation modifizieren.

4. Die Methode nach Anspruch 3, **dadurch gekennzeichnet, dass** die Knochenstrukturen und die nicht kollagenen organischen Komponenten vor der Atelopeptidation aus dem Knochenbrei entfernt werden.

5. Die Methode nach Anspruch 3 **oder 4, dadurch gekennzeichnet, dass** für die Atelopeptidation ein proteolytisches Enzym ohne jegliche kollagenolytische Aktivität verwendet wird.

6. Die Methode nach Anspruch 5, **dadurch gekennzeichnet, dass** es sich bei dem verwendeten proteolytischen Enzym um Pepsin handelt.

7. Die Methode nach den Ansprüchen 3 bis 6, **dadurch gekennzeichnet, dass** das Produkt nach der Atelopeptidation mit deionisiertem Wasser gereinigt wird, bis konstante Leitfähigkeitswerte erzielt werden, durch Sublimierung bei geringem Druck zentrifugiert, eingefroren und getrocknet wird, möglicherweise ergänzt durch den hinzugefügten Hilfsstoff, oder die Hilfsstoffe, durch Pressen in den Formen geformt wird oder eingefüllt wird und durch Gammastrahlung sterilisiert wird.

8. Das Produkt nach Anspruch 1 für die Anwendung bei der Behandlung von Knochendefekten, **dadurch gekennzeichnet, dass** das Produkt als Knochenfüllmaterial bei Knochenzysten und kleineren fehlenden Knochenteilen implantiert wird.

9. Das Produkt nach Anspruch 1 für die Anwendung bei der Behandlung von Osteoporose, Arthritis und anderen degenerativen Erkrankungen, **dadurch gekennzeichnet, dass** das Produkt als Nahrungsergänzungsmittel verabreicht wird.

## Revendications

1. Produit destiné à la facilitation de l'ossification des défauts osseux au moyen d'un composite osseux natif contenant la structure hydroxyapatite et un composant de collagène modifié, **caractérisée par le fait que** la structure hydroxyapatite comprenant la pulpe osseuse purifiée et le collagène contenus dans le composé osseux natif est modifiée et au moins partiellement dépourvue des télopeptides antigéniques terminaux.

2. Produit selon la revendication 1, **dans lequel le fait** que en plus de l'ingrédient de base, le composite osseux natif renfermant du collagène modifié contient au moins un excipient provenant du groupe des facteurs de croissance, antibiotiques, substances bactériostatiques ou pharmacologiquement actives.

3. Méthode de fabrication du produit facilitant l'ossification d'après la revendication 1, **dan**s **lequel le fait** que les enzymes sont appliquées sur la pulpe osseuse purifiée et que lesdites enzymes catalysent l'hydrolyse des télopeptides terminaux du collagène et modifient ainsi le collagène par le biais d'une atélopeptidation.

4. Méthode d'après la revendication **3, dans lequel le** fait que les structures osseuses et les composants organiques de nature non collagène sont retirés de la pulpe osseuse avant l'atélopeptidation.

5. Méthode d'après la revendication 3 ou 4, **dans lequel le fait** que une enzyme protéolytique sans activité collagénolytique est utilisée pour l'atélopeptidation.

6. Méthode d'après la revendication **5, dans lequel le fait** l'enzyme protéolytique utilisée est la pepsine.

7. Méthode selon les revendications 3 à 6**, dans lequel** 1 **le fait** que le produit issu de l'atélopeptidation est ensuite lavé à l'eau désionisée jusqu'à l'obtention de valeurs de conductivité constantes, centrifugé, congelé puis séché par sublimation à basses pressions, éventuellement complété par le ou les excipients ajoutés, mis en forme par pression dans les moules, ou rempli et stérilisé par rayonnement gamma.

8. Produit selon la revendication 1, destiné à un usage dans le traitement des défauts osseux au cours duquel le produit est implanté en tant que matériau de comblement osseux pour les kystes osseux et les petits éléments osseux manquants.

9. Produit selon la revendication 1, destiné à un usage dans le traitement de l'ostéoporose, de l'arthrite et autres maladies dégénératives, et dans lequel le produit est administré comme complément alimentaire.
